Europäisches Patentamt

European Patent Office

Office européen des brevets

(19)

(11) **EP 1 477 154 A1**

(12) **DEMANDE DE BREVET EUROPEEN**

(43) Date de publication:
**17.11.2004 Bulletin 2004/47**

(51) Int Cl.[7]: **A61K 7/043**, A61K 7/025,
A61K 7/031, A61K 7/032

(21) Numéro de dépôt: **04291242.8**

(22) Date de dépôt: **14.05.2004**

| | |
|---|---|
| (84) Etats contractants désignés:<br>**AT BE BG CH CY CZ DE DK EE ES FI FR GB GR HU IE IT LI LU MC NL PL PT RO SE SI SK TR**<br>Etats d'extension désignés:<br>**AL HR LT LV MK** | (72) Inventeurs:<br>• **Girier Duffournier, Franck**<br>**75011 Paris (FR)**<br>• **Chevalier, Gaetan**<br>**45760 Boigny sur Bionne (FR)** |
| (30) Priorité: **16.05.2003 FR 0305932** | (74) Mandataire: **Tanty, François**<br>**Nony & Associés,**<br>**3, rue de Penthièvre**<br>**75008 Paris (FR)** |
| (71) Demandeur: **L'OREAL**<br>**75008 Paris (FR)** | |

(54) **Composition cosmetique comprenant un pigment diffractant et un agent de coloration goniochromatique**

(57) La présente invention concerne une composition cosmétique, caractérisée par le fait qu'elle présente, pour un éclairage incident à +45°, une variation Dh d'angle de teinte d'au moins 50°, mieux d'au moins 70°, pour une variation de l'angle d'observation allant de -10° à +30°, et un écart $(\Delta a^{*2} + \Delta b^{*2})^{\frac{1}{2}}$ d'au moins 5 entre les angles d'observation de -30° et -70°.

FIG. 2

**Description**

La présente invention se rapporte au maquillage de la peau, par exemple celle du visage ou du corps, des lèvres ou des phanères tels que les cils, les sourcils, les ongles et les cheveux.

Il existe sur le marché de nombreuses compositions cosmétiques qui visent à créer une variation de couleur avec l'angle d'observation.

Il a notamment été incorporé dans un vernis à ongles un pigment diffractant produisant un effet arc-en-ciel, commercialisé par la société FLEX PRODUCTS sous la marque SPECTRAFLAIR, associé à un pigment interférentiel commercialisé par la même société sous la marque CHROMAFLAIR. Des pigments ayant la structure $Cr/MgF_2/Al/MgF_2/Cr$ sont commercialisés sous cette marque.

Il existe un besoin pour créer encore de nouveaux effets optiques, et notamment de nouveaux effets de variabilité de la couleur avec l'angle d'observation.

Il existe plus particulièrement un besoin pour bénéficier de compositions cosmétiques attractives et capables de présenter une variation de couleur avec l'angle d'observation pour une large plage de valeurs de l'angle d'observation, afin notamment de générer un effet visuel susceptible d'attirer le regard sous de nombreuses conditions d'éclairage et d'observation.

L'invention vise ainsi, selon un premier de ses aspects, à proposer une composition ayant une variabilité optique accrue, qui peut se caractériser par le fait qu'elle présente, pour un éclairage incident à +45°, une variation Dh de l'angle de teinte d'au moins 50°, mieux d'au moins 70°, pour une variation de l'angle d'observation allant de -10° à +30°, et un écart $(\Delta a^{*2} + \Delta b^{*2})^{\frac{1}{2}}$ d'au moins 5 entre les angles d'observation -30° et - 70°.

De préférence, la variation Dh de l'angle de teinte entre les angles d'observation -30° et -70° est supérieure ou égale à 15°, mieux supérieure ou égale à 20°.

Une telle composition peut comporter au moins un pigment diffractant. Elle peut comporter également au moins un agent de coloration goniochromatique.

De manière surprenante, la caractéristique de variation significative de la couleur de la composition pour des angles d'observation compris entre -30° et -70° peut se combiner à la caractéristique de variation significative de l'angle de teinte Dh pour des angles d'observation allant de -10° à +30°. La combinaison de ces deux caractéristiques résulte en une composition qui présente une variabilité de la couleur accrue, donc qui est susceptible de permettre à un observateur de percevoir un changement de couleur, voire un mouvement de couleur, dans de nombreuses conditions d'observation et d'illumination.

L'invention vise encore, selon un autre de ses aspects, à proposer une composition cosmétique comportant dans un milieu physiologiquement acceptable, au moins un pigment diffractant et au moins un agent de coloration goniochromatique.

Une telle association a déjà été proposée dans l'article *« Pigments Exhibiting a Combination of Thin Film and Diffractive Light Interference »*, par Alberto Argoitia, lors de la conférence AIMCAL 2002 Fall Technical Conference Meeting, Sedona, Arizona, Octobre 20-23, 2002. L'agent de coloration goniochromatique utilisé dans les compositions décrites dans cet article est constitué d'un pigment variant du vert au bleu et comportant un dépôt d'aluminium de 80 nm d'épaisseur.

Ces compositions ne permettent pas d'obtenir un effet optique complètement satisfaisant et il existe un besoin pour bénéficier d'une composition cosmétique comportant, dans un milieu physiologiquement acceptable, au moins un pigment diffractant et au moins un agent de coloration goniochromatique, qui présente une attractivité accrue, et notamment une variation de couleur sensible pour une large plage de valeurs de l'angle d'observation.

L'invention a ainsi pour objet, selon un autre de ses aspects, une composition qui peut se caractériser par le fait que l'agent de coloration goniochromatique est suffisamment transparent pour que l'on puisse observer, pour un éclairage incident à +45°, une variation Dh de l'angle de teinte de la composition d'au moins 50°, mieux d'au moins 70°, pour une variation de l'angle d'observation allant de -10° à +30° et un écart $(\Delta a^{*2} + \Delta b^{*2})^{\frac{1}{2}}$ d'au moins 5 entre les angles d'observation -30° et -70°.

Dans l'invention telle que définie ci-dessus, la variation Dh de l'angle de teinte entre les angles d'observation allant de -10° à +30° est induite essentiellement par le pigment diffractant et génère un effet arc-en-ciel, tandis que la variation de la couleur observée entre les angles -30° et -70° provient essentiellement de l'agent de coloration goniochromatique. De par la transparence suffisante de ce dernier, l'effet arc-en-ciel peut continuer à être observé malgré sa dispersion au sein de la composition comportant l'agent de coloration goniochromatique, même lorsque celui-ci est présent dans une proportion non négligeable.

Dans un exemple de mise en oeuvre avantageux de l'invention, l'agent de coloration goniochromatique ne comporte pas de dépôt métallique réfléchissant, notamment d'aluminium, mais une structure à base d'oxydes, laquelle permet d'obtenir une moindre opacité du pigment.

De préférence, l'agent de coloration goniochromatique ne comporte pas de pigment ayant une dimension supérieure à 80 μm, ce qui permet de réduire encore le risque que l'agent de coloration goniochromatique ne nuise à la perception du changement de couleur induit par le pigment diffractant.

L'agent de coloration goniochromatique peut ainsi comporter un pigment dont la dimension est inférieure ou égale à 80 μm, mieux inférieure ou égale à 70 μm, mieux encore inférieure ou égale à 50 μm, tout en étant par exemple supérieure ou égale à 4 μm, mieux supérieure ou égale à 6 μm.

Le rapport pondéral du pigment diffractant par rapport à l'agent de coloration goniochromatique est de préférence compris entre 85/15 et 15/85, mieux entre 80/20 et 20/80, mieux encore 60/40 et 40/60, par exemple de l'ordre de 50/50. Un tel rapport est favorable à l'obtention d'un effet arc-en-ciel et d'un effet goniochromatique soutenus.

L'invention a encore pour objet un vernis à ongles comportant une composition telle que définie plus haut.

L'invention a encore pour objet un produit pour le maquillage des lèvres, comportant une composition telle que définie plus haut, notamment un rouge à lèvres ou un brillant à lèvres ou gloss.

L'invention a encore pour objet un mascara comportant une composition telle que définie plus haut.

L'invention a encore pour objet un fond de teint comportant une composition telle que définie plus haut.

L'invention a encore pour objet l'utilisation d'un agent de coloration goniochromatique non opaque en association avec un pigment diffractant pour le maquillage de la peau, des lèvres ou des phanères.

L'invention a encore pour objet un procédé de maquillage d'un support choisi parmi la peau, les lèvres et les phanères, comprenant l'application simultanée sur le support d'au moins un agent de coloration goniochromatique non opaque et d'un pigment diffractant.

L'invention a encore pour objet, selon un autre de ses aspects, une composition cosmétique, notamment un vernis à ongles, comportant dans un milieu physiologiquement acceptable, entre 0,5 % et 2,5 % en poids d'un pigment diffractant de structure $MgF_2/Al/MgF_2$, mieux environ 1 %, et entre 0,5 % et 2,5 % en poids d'un agent de coloration goniochromatique de structure $Fe_2O_3/SiO_2/Fe_2O_3/SiO_2/Fe_2O_3$, mieux environ 1 %, le rapport pondéral du pigment diffractant par rapport à l'agent de coloration goniochromatique étant compris entre 85/15 et 15/85, mieux proche de 50/50, par exemple compris entre 40/60 et 60/40.

L'invention a encore pour objet, selon un autre de ses aspects, une composition cosmétique, comportant dans un milieu physiologiquement acceptable, entre 0,5 % et 2,5 % en poids d'un pigment diffractant de structure $MgF_2/Al/MgF_2$, mieux environ 1 %, et entre 0,5 % et 2,5 % en poids d'un agent de coloration goniochromatique de structure $Fe_2O_3/SiO_2/mica$-oxyde/$SiO_2/Fe_2O_3$, mieux environ 1 %, par exemple entre 0,75 et 2 %, le rapport pondéral du pigment diffractant par rapport à l'agent de coloration goniochromatique étant compris entre 85/15 et 15/85, mieux proche de 50/50, par exemple compris entre 40/60 et 60/40.

L'invention a encore pour objet, selon un autre de ses aspects, une composition cosmétique comportant dans un milieu physiologiquement acceptable, entre 0,5 % et 2,5 % en poids d'un pigment diffractant de structure $MgF_2/Al/MgF_2$, mieux 1 % environ, et entre 0,5 % et 2,5 % en poids d'un agent de coloration goniochromatique de structure $TiO_2/SiO_2/TiO_2$, mieux 1 % environ, le rapport pondéral du pigment diffractant par rapport à l'agent de coloration goniochromatique étant compris entre 85/15 et 15/85, mieux proche de 50/50, par exemple compris entre 40/60 et 60/40.

L'invention a encore pour objet, selon un autre de ses aspects, une composition cosmétique comportant dans un milieu physiologiquement acceptable, entre 0,5 % et 2,5 % en poids d'un pigment diffractant de structure $MgF_2/Al/MgF_2$, mieux 1 % environ, et entre 0,5 % et 2,5 % en poids d'un agent de coloration goniochromatique de structure $TiO_2/Al_2O_3/TiO_2$, mieux 1 % environ, le rapport pondéral du pigment diffractant par rapport à l'agent de coloration goniochromatique étant compris entre 85/15 et 15/85, mieux proche de 50/50, par exemple compris entre 40/60 et 60/40.

Par « composition cosmétique », on désigne une composition telle que définie dans la Directive 93/35 CEE du Conseil du 14 juin 1993.

Par « milieu physiologiquement acceptable », on désigne un milieu non toxique et susceptible d'être appliqué sur la peau, les lèvres ou les phanères d'êtres humains.

_Mesure de la variation de l'angle de teinte Dh et de l'écart $(\Delta a^{*2} + \Delta b^{*2})^{1/2}$_

La mesure est effectuée alors que la composition a été étalée à l'aide d'un étaleur automatique avec une épaisseur de 150 μm sur le fond noir d'une carte de contraste conventionnelle, notamment de marque ERICHSEN et de référence Typ 24/5.

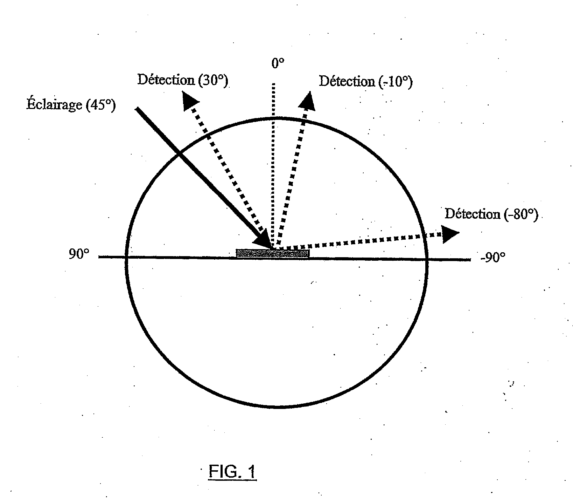

On se sert d'un spectrogonioréflectomètre, avec éclairage incident 45° et un illuminant D65, selon le schéma représenté à la figure 1. L'appareil est en mode « observateur 10° », le spectre analysé étant 400-700 nm (avec un pas 5 nm). Le spectrogonioréflectomètre utilisé est celui de marque INSTRUMENT SYSTEMS et de référence GON 360 GONIOMETER.

Un angle d'observation négatif correspond ici à une observation dans le demi plan opposé à celui d'où provient la lumière, par rapport à la normale à la surface illuminée, comme on peut le voir sur la figure 1.

Sauf précisé autrement, on suppose que dans toute la description, les mesures sont effectuées dans ces conditions d'étalement à 150 μm, après séchage de la composition.

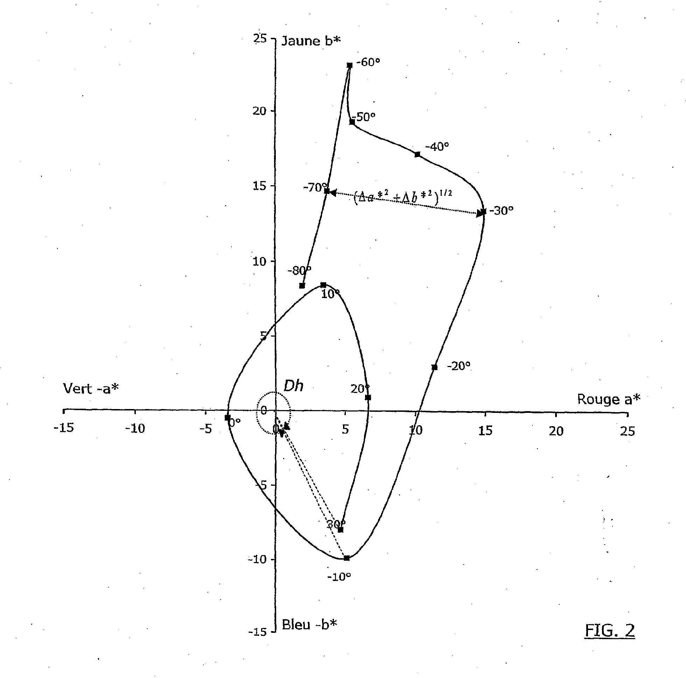

On a représenté à la figure 2, le trajet de couleur dans le plan CIE L*a*b* 1976 d'un exemple de composition selon l'invention, obtenu au moyen du spectrogonioréflectomètre utilisé comme indiqué ci-dessus.

Sur cette figure, la variation d'angle de teinte Dh est presque de 360° entre les valeurs d'angle d'observation +30° et -10°. La variation Dh correspond au secteur angulaire balayé par un rayon partant du point achromatique O et dont l'extrémité décrit le chemin de couleur, le repère étant orthonormé, c'est-à-dire l'échelle étant la même sur les axes -a*a* et -b*b*. Une variation Dh d'au moins 360° signifie que l'on traverse les quatre quadrants du plan a*b*.

L'écart $\Delta E_{ab}*$ entre les points correspondant aux angles -30° et -70° est défini par la formule :

$$\Delta E\,ab* = \sqrt{(a*_{-70°} - a*_{-30°})^2 + (b*_{-70°} - b*_{-30°})^2} = (\Delta a*^2 + \Delta b*^2)^{1/2}$$

Dans l'exemple considéré, on a :

$a*_{-70°} = 3{,}74$
$a*_{-30°} = 14{,}92$
$b*_{-70°} = 14{,}68$
$b*_{-30°} = 13{,}44$, soit
$\Delta E_{ab*} = 11{,}25$
et Dh $\approx 360°$.

L'un des points correspondant aux angles -30° et -70°, voire les deux, peut se situer dans l'un des quadrants correspondant à a*>0, comme on peut le voir sur les figures 2 à 5.

*Exemples de pigments diffractants*

Une composition selon l'invention contient un ou plusieurs pigments diffractants. On peut utiliser un seul pigment diffractant pour une facilité de mise en oeuvre.

Par « pigment diffractant », on désigne au sens de la présente invention un pigment capable de produire une variation de couleur selon l'angle d'observation lorsqu'éclairé par de la lumière blanche, en raison de la présence d'une structure qui diffracte la lumière.

Un pigment diffractant peut comporter un réseau de diffraction, capable par exemple de diffracter dans des directions définies un rayon de lumière monochromatique incident.

Le réseau de diffraction peut comporter un motif périodique, notamment une ligne, la distance entre deux motifs adjacents étant du même ordre de grandeur que la longueur d'onde de la lumière incidente.

Lorsque la lumière incidente est polychromatique, le réseau de diffraction va séparer les différentes composantes spectrales de la lumière et produire un effet arc-en-ciel.

On pourra utilement se reporter concernant la structure des pigments diffractants à l'article *« Pigments Exhibiting Diffractive Effects »* d'Alberto Argoitia and Matt Witzman, 2002, Society of Vacuum coaters, 45th Annual Technical Conference Proceedings 2002.

Le pigment diffractant peut être réalisé avec des motifs ayant différents profils, notamment triangulaires, symétriques ou non, en créneaux, de largeur constante ou non, sinuosoïdaux.

La fréquence spatiale du réseau et la profondeur des motifs seront choisies en fonction du degré de séparation des différents ordres souhaités. La fréquence peut varier par exemple entre 500 et 3000 lignes par mm.

De préférence, les particules du pigment diffractant présentent chacune une forme aplatie, et notamment sont en forme de plaquette.

Une même particule de pigment peut comporter deux réseaux de diffraction croisés, perpendiculaires ou non.

Une structure possible pour le pigment diffractant peut comporter une couche d'un matériau réfléchissant, recouverte au moins d'un côté d'une couche d'un matériau diélectrique. Ce dernier peut conférer une meilleure rigidité et durabilité au pigment diffractant. Le matériau diélectrique peut alors être choisi par exemple parmi les matériaux suivants : $MgF_2$, $SiO_2$, $AL_2O_3$, $AIF_3$, $CeF_3$, $LaF_3$, $NdF_3$, $SmF_2$, $BaF_2$, $CaF_2$, LiF et leurs associations. Le matériau réfléchissant peut être choisi par exemple parmi les métaux et leurs alliages et aussi parmi les matériaux réfléchissants non métalliques. Parmi les métaux pouvant être utilisés, on peut citer Al, Ag, Cu, Au, Pt, Sn, Ti, Pd, Ni, Co, Rd, Nb, Cr et leurs composés, associations ou alliages. Un tel matériau réfléchissant peut, seul, constituer le pigment diffractant qui sera alors monocouche.

En variante, le pigment diffractant peut comporter une structure multicouche comportant un noyau d'un matériau diélectrique recouvert d'une couche réfléchissante au moins d'un côté, voire encapsulant complètement le noyau. Une couche d'un matériau diélectrique peut également recouvrir la ou les couches réfléchissantes. Le matériau diélectrique utilisé est alors de préférence inorganique, et peut être choisi par exemple parmi les fluorures métalliques, les oxydes métalliques, les sulfures métalliques, les nitrures métalliques, les carbures métalliques et leurs associations. Le matériau diélectrique peut être à l'état cristallin, semi-cristallin ou amorphe. Le matériau diélectrique, dans cette configuration, peut par exemple être choisi parmi les matériaux suivants : $MgF_2$, SiO, $SiO_2$, $Al_2O_3$, $TiO_2$, WO, AIN, BN, $B_4C$, WC, TiC, TiN, $N_4Si_3$, ZnS, des particules de verre, des carbones de type diamant et leurs associations.

En variante, le pigment diffractant peut être composé d'un matériau diélectrique ou céramique préformé tel qu'un minéral en lamelle naturelle, par exemple du mica peroskovite ou du talc, ou des lamelles synthétiques formées à partir de verre, d'alumine, de $SiO_2$ de carbone, d'un oxyde de fer/mica, de mica recouvert de BN, de BC, de graphite, d'oxychlorure de bismuth, et leurs associations.

A la place d'une couche d'un matériau diélectrique, d'autres matériaux améliorant les propriétés mécaniques peuvent convenir. De tels matériaux peuvent comporter du silicone, des silicides métalliques, des composés semi-conducteurs formés à partir d'éléments des groupes III, IV et V, des métaux ayant une structure cristalline cubique centrée, des compositions ou composés de cermet, des verres semi-conducteurs, et leurs associations variées.

Le pigment diffractant utilisé peut notamment être choisi parmi ceux décrits dans la demande de brevet américain US 2003/0031870 publiée le 13 février 2003.

Un pigment diffractant peut comporter par exemple la structure suivante : $MgF_2/Al/MgF_2$, un pigment diffractant ayant cette structure étant commercialisé sous la dénomination SPECTRAFLAIR 1400 Pigment Silver par la société FLEX PRODUCTS, ou SPECTRAFLAIR 1400 Pigment Silver FG. La proportion en poids du $MgF_2$ peut être comprise entre 80 et 95 % du poids total du pigment.

La quantité de pigment diffractant peut varier, en poids par rapport au poids total de la composition, par exemple de 0,1 à 5 %, voire de 0,5 à 5 %, ou encore de 0,5 % à 2,5 %, par exemple de l'ordre de 1 % pour une composition destinée à être appliquée sur les ongles. Par exemple, la composition peut contenir moins de 5 % de pigment diffractant en poids par rapport au poids total de la composition, mieux entre 0,5 % et 2,5 %.

La dimension du pigment diffractant peut être comprise par exemple entre 5 et 200 μm, mieux entre 5 et 100 μm, par exemple entre 5 et 30 μm.

Par « dimension », on désigne la dimension donnée par la distribution granulométrique statistique à la moitié de la population, dite D50.

L'épaisseur des particules de pigment diffractant peut être inférieure ou égale à 3 μm, mieux 2 μm, par exemple de l'ordre de 1 μm.

Le pigment diffractant sera choisi de telle sorte que l'on puisse observer, pour un éclairage incident à 45° et une variation de l'angle d'observation comprise entre 30° et - 10°, une variation Dh de l'angle de teinte de la composition cosmétique, étalée à 150 μm comme précisé plus haut, dans le plan CIE 1976, d'au moins 50°, mieux d'au moins 70°, voire au moins 80° ou 90°, voire au moins 100°. Pour un vernis à ongles notamment, la variation Dh peut être de préférence d'au moins 180°, de préférence encore au moins 270°, voire environ 360° ou plus par exemple.

Pour un gloss, de bons résultats ont été obtenus notamment avec une variation Dh d'au moins 90°, entre les angles d'observation 30° et -10°.

*Exemples d'agents de coloration goniochromatiques*

Par « agent de coloration goniochromatique », on désigne au sens de la présente invention un agent de coloration permettant d'obtenir une variation de la couleur observée avec l'angle d'observation obtenue autrement qu'au moyen seulement d'un réseau de diffraction.

L'agent de coloration goniochromatique peut par exemple présenter une structure multicouche interférentielle.

Les demandes EP 1 195 155 et EP 1 249 226 décrivent des exemples de pigments goniochromatiques.

L'agent de coloration goniochromatique peut permettre d'obtenir, lorsque présent à 1 % en masse dans une base vernis à ongles étalée à 150 μm, une variation de l'angle de teinte Dh supérieure ou égale à 30°, voire d'au moins 60°, lorsque l'angle d'observation varie de 0° à -80°.

La composition peut contenir plusieurs agents de coloration goniochromatiques mais on peut utiliser un seul agent de coloration goniochromatique pour une facilité de mise en oeuvre.

L'agent de coloration goniochromatique peut être présent en une quantité pouvant varier, en poids par rapport au poids total de la composition, par exemple de 0,5 à 60 %, notamment selon la nature de la composition. La composition peut par exemple contenir moins de 5 % en poids de l'agent de coloration goniochromatique, mieux entre 0,5 % et 2,5 % en poids par rapport au poids total de la composition. Une composition de vernis à ongles pourra contenir par exemple entre 0,1 et 5 % d'agent de coloration goniochromatique ; un fond de teint pourra en contenir par exemple de 1 à 15 % et un rouge à lèvres pourra en contenir par exemple de 1 à 10 % en poids.

Pour un vernis à ongles, des résultats très satisfaisants ont pu être obtenus pour une teneur d'agent de coloration goniochromatique de 1 % combinée à une teneur en pigment diffractant également de 1 %, les proportions étant massiques.

L'agent de coloration goniochromatique présente de préférence une dimension inférieure à 80 μm, par exemple comprise entre 4 et 80 μm, mieux entre 6 et 70 μm.

Conformément à un aspect préféré de l'invention, l'agent de coloration goniochromatique est non opaque, c'est-à-dire suffisamment transparent pour que l'on puisse observer, pour un éclairage à 45° et une variation de l'angle d'observation comprise entre +30° et -10°, un effet arc-en-ciel se traduisant par une variation suffisante Dh de l'angle de teinte de la composition cosmétique selon l'invention, dans le plan CIE 1976. Cette variation peut être, notamment pour un vernis à ongles, de préférence d'au moins 120°, mieux d'au moins 180°, mieux encore d'au moins 270°, par exemple de l'ordre de 360°, cette variation correspondant à l'effet arc-en-ciel induit par le pigment diffractant. Pour un gloss, cette variation peut être de préférence d'au moins 90°.

L'agent de coloration goniochromatique permet également d'obtenir un écart $\Delta E_{ab}^* = (\Delta a^{*2} + \Delta b^{*2})^{\frac{1}{2}}$ de la composition cosmétique, mesuré dans l'espace colorimétrique CIE 1976, d'au moins 5, par exemple d'au moins 8, voire d'au moins 10, mieux d'au moins 15, voire 20, cet écart étant mesuré comme indiqué plus haut.

L'agent de coloration goniochromatique peut être choisi par exemple parmi les structures multicouches interférentielles non opaques et les agents de coloration à cristaux liquides non opaques.

Dans le cas d'une structure multicouche, celle-ci peut comporter par exemple au moins deux couches, chaque couche étant réalisée par exemple à partir d'au moins un matériau choisi dans le groupe constitué par les matériaux suivants : $MgF_2$, $CeF_3$, ZnS, ZnSe, Si, $SiO_2$, Ge, Te, $Fe_2O_3$, Pt, Va, $Al_2O_3$, MgO, $Y_2O_3$, $S_2O_3$, SiO, $HfO_2$, $ZrO_2$, $CeO_2$, $Nb_2O_5$, $Ta_2O_5$, $TiO_2$, Ag, Al, Au, Cu, Rb, Ti, Ta, W, Zn, $MoS_2$, cryolithe, alliages, polymères et leurs associations, dès lors qu'elle ne présente pas trop d'opacité.

Chaque couche peut être non métallique, notamment afin de ne pas nuire à la transparence de la structure.

La structure multicouche peut présenter ou non, par rapport à une couche centrale, une symétrie au niveau de la nature chimique des couches empilées. Selon l'épaisseur et la nature des différentes couches, on obtient différents effets.

Des exemples de structures multicouche interférentielles symétriques utilisables dans des compositions réalisées conformément à l'invention sont par exemple les structures suivantes : $Fe_2O_3/SiO_2/Fe_2O_3/SiO_2/Fe_2O_3$, un pigment ayant cette structure étant commercialisé sous la dénomination SICOPEARL par la société BASF ; $MoS_2/SiO_2/mica$-oxyde$/SiO_2/MoS_2$ ; $Fe_2O_3/SiO_2/mica$-oxyde$/SiO_2/Fe_2O_3$ ; $TiO_2/SiO_2/TiO_2$ et $TiO_2/Al_2O_3/TiO_2$, des pigments ayant ces structures étant commercialisés sous la dénomination XIRONA par la société MERCK (Darmstadt).

Les agents de coloration à cristaux liquides comprennent par exemple des silicones ou des éthers de cellulose sur lesquels sont greffés des groupes mésomorphes.

Comme particules goniochromatiques à cristaux liquides, on peut utiliser par exemple celles vendues par la société CHENIX ainsi que celles commercialisées sous la dénomination HELICONE® HC par la société WACKER.

Comme agent de coloration goniochromatique, on peut encore utiliser certaines nacres, des pigments à effets sur substrat synthétique, notamment substrat type alumine, silice, borosilicate, oxyde de fer, aluminium, ou des paillettes holographiques interférentielles issues d'un film de polytéréphthalate.

La composition peut en outre comporter des fibres goniochromatiques dispersées. De telles fibres présenteront de préférence une longueur inférieure à 80 μm.

*Autres agents de coloration*

La composition cosmétique peut incorporer un ou plusieurs autres agents de coloration, par exemple choisis parmi les colorants, notamment liposolubles ou hydrosolubles, les pigments monochromes, et certaines nacres classiquement utilisées dans les compositions cosmétiques.

A titre d'exemple de nacres, on peut citer le mica naturel recouvert d'oxyde de titane, d'oxyde de fer, de pigment naturel ou d'oxychlorure de bismuth. Parmi les nacres disponibles sur le marché, on peut citer les nacres TIMICA et FLAMENCO commercialisées par la société ENGELHARD et les nacres TIMIRON commercialisées par MERCK.

Un agent de coloration non goniochromatique et non diffractant peut également être choisi par exemple parmi les colorants, les pigments monochromes et peut être destiné par exemple à corriger les teintes produites par l'association décrite du pigment diffractant et de l'agent de coloration goniochromatique, de façon à éviter l'apparition d'une couleur estimée non souhaitable. Un agent de coloration non goniochromatique et non diffractant peut également être présent dans la composition cosmétique pour lui conférer une couleur souhaitée dans certaines conditions d'observation.

Le ou les agents de coloration autres que le pigment diffractant pourront être choisis de telle sorte que le chemin de couleur dans le plan a*b* CIE 1976 traverse les quatre quadrants et « entoure » en quelque sorte le point achromatique, comme on peut le voir sur la figure 2, notamment lorsque l'angle d'observation varie entre +30° et -10°.

*Milieu physiologiguement acceptable*

Le milieu physiologiquement acceptable sera adapté à la nature du support sur lequel doit être appliquée la composition ainsi qu'à la forme sous laquelle la composition est destinée à être conditionnée, notamment solide ou fluide à température ambiante et pression atmosphérique.

La composition selon l'invention peut comprendre un milieu cosmétique aqueux et/ou une phase grasse.

La composition peut comprendre de l'eau ou un mélange d'eau et de solvants organiques hydrophiles comme les alcools et notamment des monoalcools inférieurs linéaires ou ramifiés ayant de 2 à 5 atomes de carbone comme l'éthanol, l'isopropanol ou le n-propanol, les polyols comme la glycérine, la diglycérine, le propylène glycol, le sorbitol, le penthylène glycol, les polyéthylène glycols. La phase hydrophile peut, en outre, contenir des éthers en $C_2$ et des aldéhydes en $C_2$-$C_4$ hydrophiles. L'eau ou le mélange d'eau et de solvants organiques hydrophiles peut être présent dans la composition selon l'invention, en une teneur allant de 0 % à 90 % (notamment 0,1 % à 90 %) en poids, par rapport au poids total de la composition, et de préférence de 0 % à 60 % en poids (notamment 0,1 % à 60 % en poids).

La composition peut également comprendre une phase grasse, notamment constituée de corps gras liquides à température ambiante (25 °C en général) et/ou de corps gras solides à température ambiante tels que les corps gras pâteux, les gommes et leurs mélanges. Cette phase grasse peut, en outre, contenir des solvants organiques lipophiles.

Comme corps gras liquides à température ambiante, appelés souvent huiles, utilisables dans l'invention, on peut citer : les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité ; les hydrocarbures linéaires ou ramifiés, d'origine minérale ou synthétique tels que les huiles de paraffine et leurs dérivés, la vaseline, les polydécènes, le polyisobutène hydrogéné tel que le parléam ; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; des esters de polyol comme le dioctanoate de propylène glycol, le diheptanoate de néopentylglycol, le diisononanoate de diéthylèneglycol ; et les esters du, pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique ; les huiles fluorées partiellement hydrocarbonées et/ou siliconées ; les huiles siliconées comme les polyméthylsiloxanes (PDMS) volatiles ou non, linéaires ou cycliques, liquides ou pâteux à température ambiante comme les cyclométhicones, les diméthicones, comportant éventuellement un groupement phényle, comme les phényl triméthicones, les phényltriméthylsiloxydiphényl siloxanes, les diphénylméthyldiméthyl-trisiloxanes, les diphényl diméthicones, les phényl diméthicones, les polyméthylphényl siloxanes ; leurs mélanges.

Ces huiles peuvent être présentes en une teneur allant de 0,01 à 90 %, et mieux de 0,1 à 85 % en poids, par rapport au poids total de la composition.

La composition selon l'invention peut également comprendre un ou plusieurs solvants organiques, physiologiquement acceptables. Ces solvants peuvent être présents en une teneur allant de 0 à 90 %, mieux de 0 à 60 % en poids, par rapport au poids total de la composition et encore mieux de 0,1 à 30 %.

La présence de solvants organiques convient plus particulièrement pour le maquillage des ongles. La composition constitue alors généralement un vernis à ongles. Le solvant organique peut être présent dans la composition cosmétique à une teneur allant par exemple de 30 à 99 % en poids, par rapport au poids total de la composition, et de préférence de 60 % à 90 % en poids.

Lorsque le milieu physiologiquement acceptable de la composition contient une phase liquide, cette phase peut être notamment une phase organique liquide dans laquelle de l'eau est dispersée ou émulsionnée.

La composition peut présenter une phase grasse continue, pouvant contenir moins de 5 % d'eau, notamment moins de 1 % d'eau par rapport à son poids total et en particulier être sous forme anhydre.

*Charges*

La composition cosmétique peut comprendre en outre des charges.

Par « charges », on désigne des particules de toute forme insolubles dans le milieu de la composition, quelle que soit la température à laquelle la composition est fabriquée. Ces charges peuvent servir notamment à modifier la rhéologie ou la texture de la composition.

A titre d'exemple de charges, on peut citer, entre autres, le talc, le mica, la silice, le kaolin, et les poudres de polyamide (Nylon® ) (Orgasol® de chez Atochem).

Les charges et autres pigments ne doivent pas nuire aux effets optiques recherchés.

*Agents actifs cosmétiques*

La composition cosmétique peut également contenir un ou plusieurs actifs cosmétiques, dermatologiques, hygiéniques ou pharmaceutiques.

Comme actifs cosmétiques, dermatologiques, hygiéniques ou pharmaceutiques, utilisables dans les compositions de l'invention, on peut citer les hydratants (polyol comme glycérine), vitamines (C, A, E, F, B, ou PP), acides gras essentiels, huiles essentielles, céramides, sphingolipides, filtres solaires liposolubles ou sous forme de nano-particules, les actifs spécifiques de traitement de la peau (agents de protection, anti-bactériens, anti-rides...). Ces actifs peuvent être utilisés par exemple à des concentrations de 0 à 20 % et notamment de 0,001 à 15 % par rapport au poids total de la composition.

*Autres ingrédients*

La composition cosmétique peut également contenir des ingrédients couramment utilisés en cosmétique, tels que par exemple les épaississants, les tensioactifs, les oligo-éléments, les hydratants, les adoucissants, les séquestrants, les parfums, les agents alcalinisants ou acidifiants, les conservateurs, les antioxydants, les filtres UV, ou leurs mélanges.

La composition cosmétique peut, de plus, comprendre, selon le type d'application envisagée, les constituants classiquement utilisés dans les domaines considérés, qui sont présents en une quantité appropriée à la forme galénique souhaitée.

*Formes galéniques*

La composition cosmétique peut se présenter sous toute forme galénique normalement utilisée pour une application topique et notamment sous forme anhydre, sous forme d'une solution huileuse ou aqueuse, d'un gel huileux ou aqueux, d'une émulsion huile-dans-eau ou eau-dans-huile, d'une émulsion multiple, d'une dispersion d'huile dans de l'eau grâce à des vésicules situés à l'interface huile/eau.

La composition de l'invention peut être sous forme de poudre, de liquide, de solide ou de semi-solide, notamment de produit coulé en stick ou en coupelle, de bâtonnet, de pâte ou de crème plus ou moins fluide, d'une couche déposée sur une feuille souple.

La composition cosmétique peut constituer, entre autres, un vernis à ongles, un rouge à lèvres, un gloss liquide, une pâte de rouge à lèvres, un fard à joues, un crayon à lèvres, un fond de teint solide ou fluide, un produit anti-cernes ou de contour des yeux, un eye-liner, un mascara, une ombre à paupières, un produit de maquillage du corps ou des cheveux ou encore un produit solaire ou de coloration de la peau.

La composition de l'invention peut être obtenue selon les procédés de préparation classiquement utilisés en cosmétique.

Les exemples soumis ci-après sont présentés à titre illustratif et non limitatif de l'invention.

Exemple 1

Vernis à ongles

On a réalisé un vernis à ongles ayant la formulation suivante (proportions en masse) :

- Pigment diffractant* 1 %
- Agent de coloration goniochromatique** 1 %
- Base vernis à ongles*** q.s.p. 100 %

  *SPECTRAFLAIR 1400 SILVER (FLEX PRODUCTS)
  **SICOPEARL (BASF)
  ***La base vernis à ongles présente la composition suivante (%) :

- Nitrocellulose 10
- Plastifiants et résine 15
- Agent rhéologique 1,5
- Acétate d'éthyle, acétate de butyle qsp 100 %

  On obtient une composition présentant le chemin de couleur représenté à la figure 2.

  On peut constater que l'on observe pour une variation de l'angle d'observation comprise entre 30° et -10° une variation Dh de l'angle de teinte d'environ 360° et un écart $\Delta E_{ab}^{*}$ supérieur à 5.

  Avec un tel vernis, on obtient une variabilité importante de la couleur pour une large plage de valeurs de l'angle d'observation, et la présence du pigment goniochromatique ne suit pas à l'observation d'un effet arc-en-ciel.

Exemple 2

Vernis à ongles

- Pigment diffractant*      1 %
- Agent de coloration goniochromatique**      1 %
- Base vernis à ongles***      qsp 100 %

    *SPECTRAFLAIR 1400 SILVER (FLEX PRODUCTS)
    ** XIRONA MAGIC MAUVE (MERCK)
    ***La base vernis à ongles présente la composition suivante (%) :

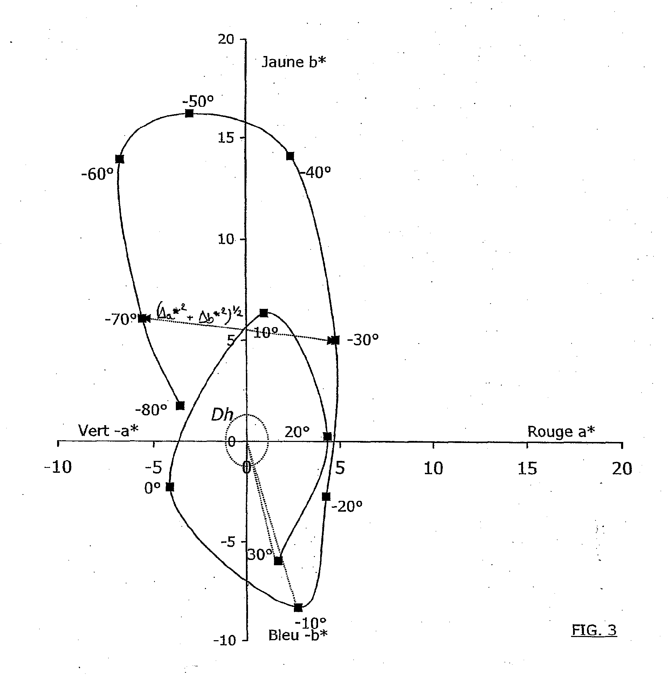

- Nitrocellulose      10
- Plastifiants et résine      15
- Agent rhéologique      1,5
- Acétate d'éthyle, acétate de butyle      qsq 100 %
    On observe le trajet de couleur représenté à la figure 3, et une forte variation de la couleur avec l'angle d'observation.

Exemple 3

Vernis à ongles

- Pigment diffractant*      1 %
- Agent de coloration goniochromatique**      1 %
- Base vernis à ongles***      qsp 100 %

    *SPECTRAFLAIR 1400 SILVER (FLEX PRODUCTS)
    ** XIRONA CARIBBEAN BLUE (MERCK)
    ***La base vernis à ongles présente la composition suivante (%):

- Nitrocellulose      10
- Plastifiants et résine      15
- Agent rhéologique      1,5
- Acétate d'éthyle, acétate de butyle      qsp 100 %

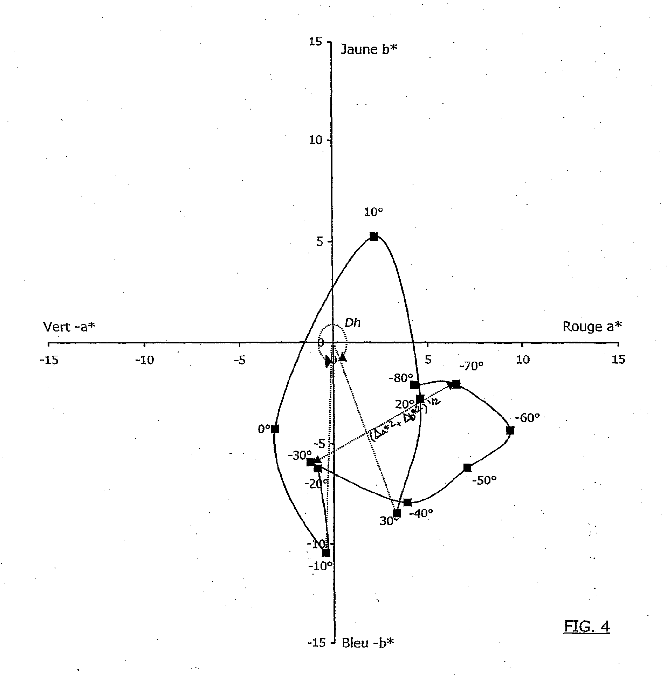

    On observe le trajet de couleur de la figure 4.

Exemple 4

Gloss

- Pigment diffractant*      1 %
- Agent de coloration goniochromatique**      1 %
- Base gloss***      qsp 100 %

    *SPECTRAFLAIR 1400 SILVER (FLEX PRODUCTS)
    **SICOPEARL (BASF)
    * * *La base gloss présente la composition suivante (%) :

- Polyacryladipate-2 de bis-diglycéryle 19,4
- Malate de diisostéraryle      10,5

- Trimellitate de tridécyle     11,1
- Triglycéride d'acide $C_{18-36}$     21,1
- Silice diméthyl silylate     8,9
- Polybutène     13,3
- Tétra isostéarate de pentaerythrityle     14,4
- Parfum, conservateur     q.s

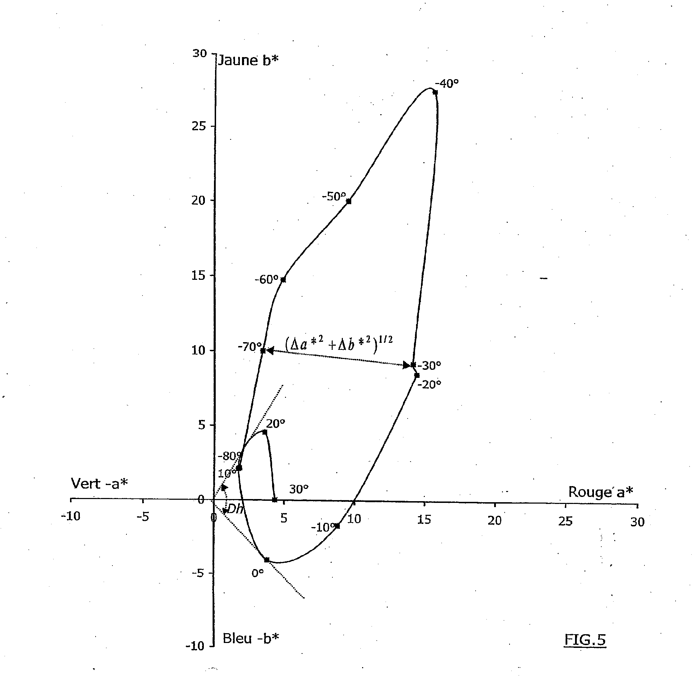

On observe le trajet de couleur de la figure 5.

On peut mesurer une variation Dh de l'angle de teinte d'environ 100° lorsque l'angle d'observation varie de 30° à -10°.

Bien entendu, l'invention n'est pas limitée aux exemples qui viennent d'être donnés, et d'autres pigments peuvent encore être utilisés.

Dans toute la description, y compris les revendications, l'expression « comportant un » doit être comprise comme étant synonyme de « comportant au moins un », sauf si le contraire est spécifié. Les intervalles doivent se comprendre bornes incluses, sauf si le contraire est spécifié.

**Revendications**

1. Composition cosmétique, **caractérisée par le fait qu'**elle présente, pour un éclairage incident à +45°, une variation Dh d'angle de teinte d'au moins 50°, mieux d'au moins 70°, pour une variation de l'angle d'observation allant de -10° à +30°, et un écart $(\Delta a^{*2} + \Delta b^{*2})^{\frac{1}{2}}$ d'au moins 5 entre les angles d'observation de -30° et -70°.

2. Composition selon la revendication 1, **caractérisée par le fait qu'**elle comporte dans un milieu physiologiquement acceptable, au moins un pigment diffractant.

3. Composition selon l'une des revendications 1 et 2, **caractérisée par le fait qu'**elle comporte au moins un agent de coloration goniochromatique, suffisamment transparent pour que l'on puisse observer ladite variation Dh de l'angle de teinte.

4. Composition selon la revendication précédente, **caractérisée par le fait que** l'agent de coloration goniochromatique comporte un pigment ayant une dimension inférieure ou égale à 80 µm, mieux comprise entre 6 et 70 µm.

5. Composition selon l'une quelconque des revendications précédentes, **caractérisée par le fait que** la variation d'angle de teinte Dh est supérieure ou égale à 80°, pour une variation de l'angle d'observation comprise entre +30° et -10°, mieux supérieure ou égale à 90°, voire 100°, et pour un vernis à ongles, notamment supérieure ou égale à 180°, mieux encore supérieure ou égale à 270°, notamment d'environ 360°, pour une variation de l'angle d'observation comprise entre +30° et -10°.

6. Composition selon l'une quelconque des revendications précédentes, **caractérisée par le fait que** l'écart $(\Delta a^{*2} + \Delta b^{*2})^{\frac{1}{2}}$ est d'au moins 8, mieux au moins 10, mieux encore au moins 15, voire 20, entre les angles d'observation -30° et -70°.

7. Composition selon l'une quelconque des revendications précédentes, **caractérisée par le fait que** la variation Dh de l'angle de teinte entre les angles d'observation -30° et -70° est supérieure ou égale à 15°.

8. Composition selon l'une quelconque des revendications précédentes, **caractérisée par le fait que** le rapport pondéral du pigment diffractant par rapport à l'agent de coloration goniochromatique est compris entre 85/15 et 15/85, mieux entre 80/20 et 20/80, mieux encore 60/40 et 40/60, par exemple 50/50 environ.

9. Composition selon l'une quelconque des revendications précédentes, **caractérisée par le fait qu'**elle comporte moins de 5 % en poids par rapport au poids total de la composition d'un pigment diffractant, mieux entre 0,5 % et 2,5 % d'un pigment diffractant.

10. Composition selon l'une quelconque des revendications précédentes, **caractérisée par le fait qu'**elle comporte moins de 5 % en poids d'un agent de coloration goniochromatique, mieux entre 0,5 % et 2,5 % d'agent de coloration goniochromatique par rapport au poids total de la composition.

**11.** Composition selon l'une quelconque des revendications précédentes, **caractérisée par le fait qu'**elle comporte au moins un pigment diffractant comportant une couche d'un matériau réfléchissant recouverte au moins d'un côté d'une couche d'un matériau diélectrique.

**12.** Composition selon la revendication précédente, **caractérisée par le fait que** le matériau réfléchissant est choisi parmi les métaux suivants : Al, Ag, Cu, Au, Pt, Sn, Ti, Pd, Ni, Co, Rd, Nb, Cr et leurs associations ou alliages, et **par le fait que** le matériau diélectrique est choisi parmi les suivants : $MgF_2$, $SiO_2$, $Al_2O_3$, $AlF_3$, $CeF_3$, $LaF_3$, $NdF_3$, $SmF_2$, $BaF_2$, $CaF_2$, LiF et leurs associations.

**13.** Composition selon l'une quelconque des revendications 1 à 10, **caractérisée par le fait qu'**elle comporte au moins un pigment diffractant comportant une couche d'un matériau diélectrique recouverte par une couche réfléchissante au moins d'un côté.

**14.** Composition selon la revendication précédente, **caractérisée par le fait que** le matériau diélectrique est choisi parmi les suivants : $MgF_2$, SiO, $SiO_2$, $Al_2O_3$, $TiO_2$, WO, AIN, BN, $B_4C$, WC, TiC, TiN, $N_4Si_3$, ZnS, des particules de verre, des carbones de type diamant et leurs associations.

**15.** Composition selon l'une quelconque des revendications précédentes, **caractérisée par le fait qu'**elle comporte au moins un agent de coloration goniochromatique comportant au moins une couche d'un matériau choisi parmi les suivants : $MgF_2$, $CeF_3$, ZnS, ZnSe, Si, $SiO_2$, Ge, Te, $Fe_2O_3$, Pt, Va, $Al_2O_3$, MgO, $Y_2O_3$, $S_2O_3$, SiO, $HfO_2$, $ZrO_2$, $CeO_2$, $Nb_2O_5$, $Ta_2O_5$, $TiO_2$, Ag, Al, Au, Cu, Rb, Ti, Ta, W, Zn, $MoS_2$, cryolithe, alliages, polymères et leurs associations.

**16.** Composition selon l'une quelconque des revendications 1 à 15, **caractérisée par le fait qu'**elle est dépourvue d'agent de coloration goniochromatique comportant une couche d'un métal réfléchissant, notamment une couche d'aluminium.

**17.** Composition selon l'une quelconque des revendications précédentes, **caractérisée par le fait qu'**elle comporte au moins un agent de coloration non goniochromatique.

**18.** Composition selon la revendication précédente, **caractérisée par le fait que** l'agent de coloration non goniochromatique est choisi parmi les colorants et les pigments monochromes.

**19.** Composition selon l'une quelconque des revendications précédentes, **caractérisée par le fait qu'**elle se présente sous l'une des formes suivantes : sous forme anhydre, sous forme d'une solution huileuse ou aqueuse, d'un gel huileux ou aqueux, d'une émulsion huile-dans-eau ou eau-dans-l'huile, d'une émulsion multiple, d'une dispersion d'huile dans de l'eau grâce à des vésicules situées à l'interface huile-eau.

**20.** Vernis à ongles comportant une composition telle que définie dans l'une quelconque des revendications précédentes.

**21.** Produit pour le maquillage des lèvres comportant une composition telle que définie dans l'une des revendications 1 à 19.

**22.** Mascara comportant une composition telle que définie dans l'une quelconque des revendications 1 à 19.

**23.** Fond de teint comportant une composition telle que définie dans l'une quelconque des revendications 1 à 19.

**24.** Utilisation d'un agent de coloration goniochromatique non opaque en association avec un pigment diffractant pour le maquillage de la peau, des lèvres ou des phanères.

**25.** Procédé de maquillage d'un support choisi parmi la peau, les lèvres et les phanères, comprenant l'application simultanée sur le support d'au moins un agent de coloration goniochromatique non opaque et d'un pigment diffractant.

**26.** Procédé selon la revendication précédente, **caractérisé par le fait que** l'agent de coloration goniochromatique et le pigment diffractant sont appliqués simultanément sous la forme d'une composition telle que définie dans l'une quelconque des revendications 1 à 19.

**27.** Composition cosmétique, **caractérisée par le fait qu'**elle comporte, dans un milieu physiologiquement acceptable, entre 0,5 % et 2,5 % en poids d'un pigment diffractant de structure $MgF_2/Al/MgF_2$, mieux 1 % environ, et entre 0,5 % et 2,5 % en poids d'un agent de coloration goniochromatique de structure $Fe_2O_3/SiO_2/Fe_2O_3/SiO_2/Fe_2O_3$, mieux 1 % environ, le rapport pondéral du pigment diffractant par rapport à l'agent de coloration goniochromatique étant compris entre 85/15 et 15/85, mieux proche de 50/50.

**28.** Composition cosmétique, **caractérisée par le fait qu'**elle comporte, dans un milieu physiologiquement acceptable, entre 0,5 % et 2,5 % en poids d'un pigment diffractant de structure $MgF_2/Al/MgF_2$, mieux 1 % environ, et entre 0,5 % et 2,5 % en poids d'un agent de coloration goniochromatique de structure $Fe_2O_3/SiO_2/mica$-oxyde$/SiO_2/Fe_2O_3$, mieux 1 % environ, le rapport pondéral du pigment diffractant par rapport à l'agent de coloration goniochromatique étant compris entre 85/15 et 15/85, mieux proche de 50/50.

**29.** Composition cosmétique, **caractérisée par le fait qu'**elle comporte, dans un milieu physiologiquement acceptable, entre 0,5 % et 2,5 % en poids d'un pigment diffractant de structure $MgF_2/Al/MgF_2$, mieux 1 % environ, et entre 0,5 % et 2,5 % en poids d'un agent de coloration goniochromatique de structure $TiO_2/SiO_2/TiO_2$, mieux 1 % environ, le rapport pondéral du pigment diffractant par rapport à l'agent de coloration goniochromatique étant compris entre 85/15 et 15/85, mieux proche de 50/50.

**30.** Composition cosmétique, **caractérisée par le fait qu'**elle comporte, dans un milieu physiologiquement acceptable, entre 0,5 % et 2,5 % en poids d'un pigment diffractant de structure $MgF_2/Al/MgF_2$, mieux 1 % environ, et entre 0,5 % et 2,5 % en poids d'un agent de coloration goniochromatique de structure $TiO_2/Al_2O_3/TiO_2$, mieux 1 % environ, le rapport pondéral du pigment diffractant par rapport à l'agent de coloration goniochromatique étant compris entre 85/15 et 15/85, mieux proche de 50/50.

FIG. 1

FIG. 2

FIG. 3

EP 1 477 154 A1

FIG. 4

FIG.5

**Office européen**
**des brevets**

## RAPPORT PARTIEL
## DE RECHERCHE EUROPEENNE
qui selon la règle 45 de la Convention sur le brevet
européen est consideré, aux fins de la procédure ultérieure,
comme le rapport de la recherche européenne

Numéro de la demande

EP 04 29 1242

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| Y,D | EP 1 195 155 A (OREAL) 10 avril 2002 (2002-04-10) * exemple 4 * | 27-30 | A61K7/043 A61K7/025 A61K7/031 A61K7/032 |
| Y | WO 03/011980 A (FLEX PRODUCTS INC) 13 février 2003 (2003-02-13) * revendication 45 * | 27-30 | |
| A | US 2003/051634 A1 (TAKAHASHI NORIO) 20 mars 2003 (2003-03-20) * alinéa [0115] * | | |
| A | WO 01/51015 A (COLOR ACCESS INC) 19 juillet 2001 (2001-07-19) * page 3, ligne 16 - ligne 21; exemple 1 * | | |
| A | PATENT ABSTRACTS OF JAPAN vol. 2002, no. 11, 6 novembre 2002 (2002-11-06) & JP 2002 212032 A (SHISEIDO CO LTD), 31 juillet 2002 (2002-07-31) * abrégé * | | |

**DOMAINES TECHNIQUES RECHERCHES (Int.Cl.7)**

A61K

### RECHERCHE INCOMPLETE

La division de la recherche estime que la présente demande de brevet, ou une ou plusieurs revendications, ne sont pas conformes aux dispositions de la CBE au point qu'une recherche significative sur l'état de la technique ne peut être effectuée, ou seulement partiellement, au regard de ces revendications.

Revendications ayant fait l'objet d'une recherche complète:

Revendications ayant fait l'objet d'une recherche incomplète:

Revendications n'ayant pas fait l'objet d'une recherche:

Raison pour la limitation de la recherche:

voir feuille supplémentaire C

| Lieu de la recherche | Date d'achèvement de la recherche | Examinateur |
|---|---|---|
| Munich | 6 septembre 2004 | Werner, S |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C08)

Office européen
des brevets

**RECHERCHE INCOMPLETE
FEUILLE SUPPLEMENTAIRE C**

Numéro de la demande

EP 04 29 1242

Revendications ayant fait l'objet de recherches complètes:
27-30

Revendications n'ayant pas fait l'objet de recherches:
1-26

Raison pour la limitation de la recherche:

La présente revendication 1 a trait à un produit défini uniquement au moyen des paramètres suivants:
P1: Dh
P2: écart
mésuré dans des conditions spécifiques.

L'utilisation de ces paramètres est considérée, dans le présent contexte, comme menant à un manque de clarté au sens de l'Article 84 CBE. Il est impossible de comparer les paramètres que le déposant a choisi d'utiliser avec ce qui est révélé dans l'état de la technique. Le manque de clarté qui en découle est tel qu'une recherche significative sur l'ensemble de l'étendue de la revendication 1 est impossible.

Les produits de la présente revendication 8, l'utilisation de la présente revendication 24 et le procédé de la présente revendication 25 ont trait à une combinaison de deux pigments goniochromatiques, selon les présentes revendications 3, 16, 24 et 25, l'un des pigments étant transparent.

La différence entre ces pigments est obscure. La seule explication se trouve dans la description à la page 9, lignes 14-15: "...obtenue autrement qu'au moyen seulement d'un reseau de diffraction". Cette explication manque de clarté et/ou de concision au sens de l'Article 84 CBE.

Par conséquent, la recherche a été effectuée pour les parties de la demande qui apparaissent être claires et/ou concises, c'est à dire les revendications 27-30.

**ANNEXE AU RAPPORT DE RECHERCHE EUROPEENNE
RELATIF A LA DEMANDE DE BREVET EUROPEEN NO.**
EP 04 29 1242

La présente annexe indique les membres de la famille de brevets relatifs aux documents brevets cités dans le rapport de recherche européenne visé ci-dessus.
Lesdits membres sont contenus au fichier informatique de l'Office européen des brevets à la date du
Les renseignements fournis sont donnés à titre indicatif et n'engagent pas la responsabilité de l'Office européen des brevets.

06-09-2004

| Document brevet cité au rapport de recherche | | Date de publication | Membre(s) de la famille de brevet(s) | | Date de publication |
|---|---|---|---|---|---|
| EP 1195155 | A | 10-04-2002 | FR | 2814672 A1 | 05-04-2002 |
| | | | EP | 1195155 A2 | 10-04-2002 |
| | | | JP | 2002154927 A | 28-05-2002 |
| | | | US | 2002064509 A1 | 30-05-2002 |
| WO 03011980 | A | 13-02-2003 | US | 2003031870 A1 | 13-02-2003 |
| | | | CA | 2449992 A1 | 13-02-2003 |
| | | | EP | 1414913 A1 | 06-05-2004 |
| | | | WO | 03011980 A1 | 13-02-2003 |
| | | | US | 2003104206 A1 | 05-06-2003 |
| US 2003051634 | A1 | 20-03-2003 | JP | 2003089758 A | 28-03-2003 |
| WO 0151015 | A | 19-07-2001 | US | 6428773 B1 | 06-08-2002 |
| | | | AU | 2639301 A | 24-07-2001 |
| | | | CA | 2366626 A1 | 19-07-2001 |
| | | | EP | 1189586 A2 | 27-03-2002 |
| | | | JP | 2003519644 T | 24-06-2003 |
| | | | WO | 0151015 A2 | 19-07-2001 |
| JP 2002212032 | A | 31-07-2002 | AUCUN | | |

EPO FORM P0460

Pour tout renseignement concernant cette annexe : voir Journal Officiel de l'Office européen des brevets, No.12/82